# FASCICULE DE BREVET EUROPEEN

(11) **EP 0 392 929 B1**
(45) Date de publication et mention de la délivrance du brevet: **12.01.1994**
(21) Numéro de dépôt: 90401019.6
(22) Date de dépôt: 12.04.1990
(51) Int. Cl.: C07D 263/28, A61K 31/42

(54) **Nouvelles amino-2-aryloxyméthyl-5 oxazolines et leurs sels**
2-Amino-5-aryloxymethyl-5-oxazolin-Derivate und ihre Salze
2-Amino-5-aryloxymethyl-5-oxazoline derivatives and salts

(30) Priorité: 14.04.1989 FR 8905180
(43) Date de publication de la demande: 17.10.1990
(73) Titulaire: LABORATOIRES SARGET, 33701 Merignac Cédex (FR)
(72) Inventeur: Descas, Patrick, F-33320 Eysines (FR); Panconi, Emmanuel, Résidence Beaurepère, F-33700 Merignac (FR); Jarry, Christian, F-33370 Artigues (FR)
(74) Mandataire: Phélip, Bruno

(56) Documents cités:
- US-A- 3 637 726
- US-A- 3 818 028
- BULLETIN DE LA SOCIETE PHARMACEUTIQUE DE BORDEAUX, vol. 120, 1981, pages 153-162; C. JARRY et al.: "Synthèse et étude structurale d'amino-2 oxazolines-2 à action antihypertensive"
- JOURNAL OF MEDICINAL CHEMISTRY, vol. 6, no. 3, mai 1963, pages 266-271, Washington, DC, US; G.I. POOS et al.: "2-amino-5-aryl-2-oxazolines. Potent new anorectic agents"

## Description

La présente invention concerne de nouvelles amino-2 aryloxyméthyl-5 oxazolines ainsi que leurs sels avec des acides pharmacologiquement compatibles.

Les composés de l'invention ont pour formule générale 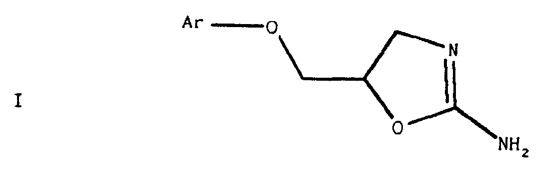
dans laquelle Ar est un groupe phényl-2 phényl, phényl-3 phényl ou phényl-4 phényl.

Les composés de l'invention sont préparés par condensation des aryloxy-1 époxy-2,3 propanes de structure II 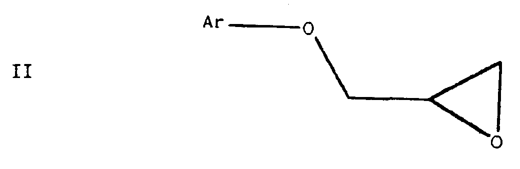
dans laquelle Ar prend les mêmes valeurs que précédemment avec du mono cyanamide de sodium dans un solvant organique soluble dans l'eau tel que les alcools inférieurs de C₁ à C₄ comme le le méthanol, l'éthanol, l'isopropanol ou le butanol, ou le tétrahydrofuranne ou le diméthylformamide et à une température comprise entre 0° et 80°.

Les composés de structure II sont connus, et leurs préparations sont décrites dans le brevet US2181085 de la société Dow Chemical Company.

La demanderesse a déja décrit dans les brevets FR2533922 et FR2546167 des amino-2 aminométhyl-5 oxazolines.

JARRY et coll a décrit Bull. Soc. Pharm. Bordeaux, 1981, 120, 153-162 des aryloxyméthyl-5 amino-2 oxazolines de structure III, 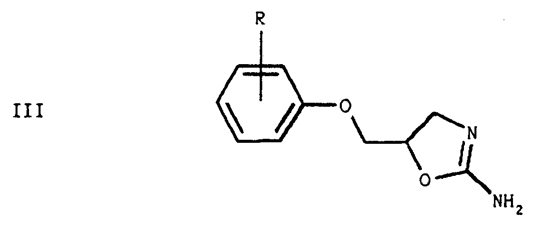
où le groupe R est un groupe méthyl en 4, un chlore en 4, un méthoxy en 4, un groupe acetamido en 4, ou allyl en 4 et méthoxy en 2, le groupe R est un groupe CH=CH-CH=CH en 2,3 et constitue avec le cycle benzénique un groupe naphtyl-1. Ces composés possèdent des propriétés antihypertensives intéressantes.

La société DOW CHEMICAL a décrit dans le brevet US3637726 des (dihalo-3,4 phénoxy)méthyl-5 amino-2 oxazolines de structure IV 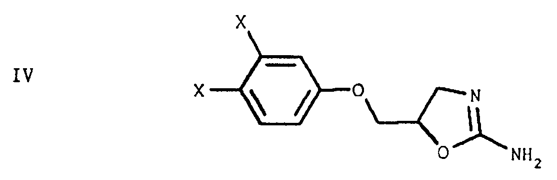
X représente un atome d'halogène chlore, brome. Ces composés possédent d'intéressantes propriétés antimicrobiennes.

Le brevet US-A-3818028 décrit des(trihalo-2,4,6 phénoxy) méthyl-5 amino-2 oxazolines utiles comme antidépresseurs.

L'invention va être décrite de façon plus précise dans les exemples suivants

Les points de fusion sont pris au banc Kofler, les déplacements chimiques sont exprimés en ppm par rapport au TMS utilisé comme étalon interne.

### EXEMPLE 1

Amino-2 (phényl-2 phénoxyméthyl)-5 oxazoline ou COR3264, produit de structure 1 avec Ar= phényl-2 phényl.

### SYNTHESE

Dans un ballon de 500 cm³, muni d'un agitateur et d'un réfrigérant on introduit 34 g de (phényl-2 phénoxy)-1 époxy-2,3 propane, 150 ml de méthanol anhydre et 11 g de monocyanamide de sodium. On agite le mélange réactionel pendant 16 heures à température ambiante. On filtre sur Célite 535 les sels minéraux. Le filtrat est évaporé sous vide. Le résidu est repris par 250 ml de chloroforme. La solution chloroformique est lavée avec deux fois 20 ml d'eau, séchée sur sulfate de sodium et évaporée sous vide. Le produit obtenu est recristallisé dans de l'heptane. On obtient 13 g de COR3264 sous forme d'un solide blanc. Rendement=31%, masse molaire=268,32.

### PROPRIETES PHYSICOCHIMIQUES

Point de fusion 124°

Spectre de RMN dans CDCl₃

3,2-4,2 ppm, 4 protons, massif complexe, 2 CH₂; 4,5-5,0 ppm, 3 protons, massif complexe, 1 CHO de l'oxazoline + NH₂ échangeable avec D₂O; 6,8-7,7 ppm, 9 protons, massif complexe, protons aromatiques.

### EXEMPLE 2

Amino-2 (phényl-4 phénoxyméthyl)-5 oxazoline ou COR4408, produit de structure 1 avec Ar= phényl-4 phényl.

### SYNTHESE

Dans un ballon de 1500 cm³, muni d'un agitateur et d'un réfrigérant on introduit 55 g de (phényl-4 phénoxy)-1 époxy-2,3 propane, dissout dans 800 ml d'un mélange tétrahydrofuranne méthanol 50/50 puis on ajoute 32 g de monocyanamide de sodium. On agite le mélange réactionel pendant 48 heures à température ambiante. On filtre sur Célite 535 les sels minéraux. Le filtrat est évaporé sous vide. Le résidu est repris par 250 ml d'éther éthylique, le solide qui a précipité est filtré puis lavé par 200 ml d'eau. Le solide obtenu est recristallisé dans un mélange toluène méthanol et séché sous vide. On obtient 30,5 g de COR4408 sous forme d'un solide blanc. Rendement=47%, masse molaire=268,32.

### PROPRIETES PHYSICOCHIMIQUES

Point de fusion 201°

Spectre de RMN dans le DMSO D₆

3,1-4,3 ppm, 4 protons, massif complexe, 2 CH₂; 4,5-5,0 ppm, 1 proton, multiplet, 1 CHO de l'oxazoline; 5,8 ppm, pic large, 2 protons, NH₂, échangeable avec D₂O; 6,9-7,7 ppm, 9 protons, massif complexe, protons aromatiques.

### EXEMPLE 3

Amino-2 (phényl-3 phénoxyméthyl)-5 oxazoline ou COR4463, produit de structure 1 avec Ar= phényl-3 phényl.

### SYNTHESE

Dans un ballon de 500 cm³, muni d'un agitateur et d'un réfrigérant on introduit 18 g de mono cyanamide de sodium dans 150 ml de méthanol anhydre puis on ajoute goutte à goutte 28 g de (phényl-3 phénoxy)-1 époxy-2,3 propane dissout dans 50 ml de méthanol anhydre. On agite le mélange réactionel pendant 16 heures à température ambiante. On filtre sur Célite 535 les sels minéraux. Le filtrat est évaporé sous vide. Le résidu est repris par 250 ml de dichlorométhane. La solution est lavée avec deux fois 20 ml d'eau, séchée sur sulfate de sodium et évaporée sous vide. Le produit obtenu est recristallisé dans de l'heptane. On obtient 15 g de COR4463 sous forme d'un solide blanc. Rendement=48%, masse molaire=268,32.

### PROPRIETES PHYSICOCHIMIQUES

Point de fusion 124°

Spectre de RMN dans CDCl₃

3,3-4,3 ppm, 4 protons, massif complexe, 2 CH₂; 4,6-5,3 ppm, 3 protons, massif complexe, 1 CHO de l'oxazoline + NH₂ échangeable avec D₂O; 6,7-7,7 ppm, 9 protons, massif complexe, protons aromatiques.

### PROPRIETES TOXICOLOGIQUES

La toxicité aigu-e des COR3264, COR44O8 et COR4463 a été determinée chez la souris après administration par voie orale. Les DL50 pour ces trois composés sont données ci dessous :
1. COR3264 DL50= 273 mg/kg
2. COR4408 DL50= 800 mg/kg
3. COR4463 DL50= 389 mg/kg

### PROPRIETES PHARMACOLOGIQUES

Les composés de l'invention ont été testés dans un screening psychopharmacologique. Les différents tests utilisés sont énumérés ci dessous.
· Mesure de l'activité motrice en actimètre de Boissier.
· Antagonisme vis à vis de l'hypothermie provoquée par l'injection sous cutanée de 2,5 mg/kg de réserpine.
· Antagonisme vis à vis de l'hypothermie provoquée par l'injection sous cutanée de 16 mg/kg d'apomorphine.
· Potentiation de la toxicité par la yohimbine
· TST, test de suspension par la queue.

Tous les produits ont été administrés par voie orale.

Dans le test d'actimétrie, le COR3264 ne présente aucune activité jusqu'à la dose de 64 mg/kg et entraîne une légére diminution de l'activité motrice spontanée à la dose de 128 mg/kg, le COR4408 ne présente aucune activité jusqu'à la dose de 64 mg/kg et entraîne une diminution de l'activité motrice spontanée à la dose de 256 mg/kg, le COR4463 est sédatif dès la dose de 16 mg/kg.

L'activité vis à vis de l'hypothermie induite par la réserpine a été déterminée chez la souris, le COR3264 ne présente aucune activité jusqu'à la dose de 128 mg/kg, le COR4408 a une DE50 de 40 mg/kg, le COR4463 est inactif jusqu'à la dose de 128 mg/kg

L'activité vis à vis de l'hypothermie induite part l'injection de 16 mg/kg d'apomorphine a été déterminée chez la souris, le COR3264 ne présente aucune activité jusqu'à la dose de 128 mg/kg, le COR4408 présente une DE50 de 73 mg/kg, le COR4463 est inactif jusqu'à la dose de 128 mg/kg

Dans le test de suspension par la queue une dose efficace 25 ,DE25, a été déterminée pour les trois substances, pour le COR3264 elle est de 16 mg/kg, pour le COR4408 elle est de 130 mg/kg et pour le COR4463 elle est de 90 mg/kg.

La potentiation de la toxicité par la yohimbine a été déterminée. Les DL50 après administration de 30 mg/kg de yohimbine par voie sous cutanée sont pour le COR3264 de 70 mg/kg, le COR4408 de 16 mg/kg et le COR4463 de 37 mg/kg.

Les résultats pharmacologiques, décrits ci dessus, sont prédictifs d'une activité antidépressive pour les composés de l'invention.

Compte tenu de leurs activités toxicopharmocologiques les produits faisant l'objet de la présente invention sont donc utiles, seuls ou associés à d'autres principes actifs, dans le traitement des états dépressifs.

Les doses et les schémas thérapeutiques seront fonction du sujet et de l'affection à traiter. Les produits pourront être administrés par voie orale ( par exemple sous forme de gélules, comprimés, sirop, gouttes buvables ), par voie injectable ( soluté injectable pour la voie intramusculaire ou la voie intraveineuse, soluté pour la perfusion intraveineuse ), par voie rectale ( suppositoires ), par voie locale ( crèmes, pommades, gels ). Suivant les indications la dose quotidienne variera de 10 à 2000 mg par jour répartie en une à quatre prises.

## Revendications (Revendications pour l'(les) Etat(s) contractant(s) suivant(s): AT, BE, CH, DE, DK, FR, GB, IT, LI, LU, NL, SE)

1. Nouveaux produits de formule générale 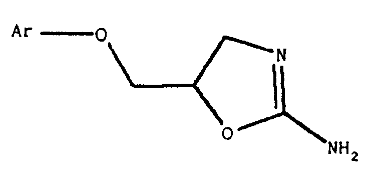 avec Ar = phényl-2 phényl ou phényl-3 phényl ou phényl-4 phényl.

2. Méthode de préparation des produits selon la revendication 1 caractérisée en ce que l'on condense dans un solvant organique soluble dans l'eau tel que les alcools inférieurs, le tétrahydrofuranne, le diméthylformamide et à une température comprise entre 0° et 80°, le dérivé mono sodé du cyanamide sur un aryloxy-1 époxy-2,3 propane de formule 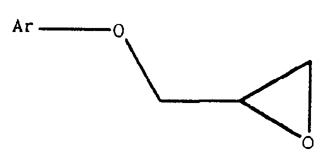 avec Ar = phényl-2 phényl ou phényl-3 phényl ou phényl-4 phényl.

3. Nouveau médicament caractérisé en ce que le principe actif est constitué par au moins un des produits selon la revendication 1

4. Nouveau médicament, selon la revendication 3, caractérisé en ce que le principe actif est constitué par l'amino-2 (phényl-2 phénoxyméthyl)-5 oxazoline ou COR3264.

5. Nouveau médicament, selon la revendication 3, caractérisé en ce que le principe actif est constitué par l'amino-2 (phényl-4 phénoxyméthyl)-5 oxazoline ou COR4408.

6. Nouveau médicament, selon la revendication 3, caractérisé en ce que le principe actif est constitué par l'amino-2 (phényl-3 phénoxyméthyl)-5 oxazoline ou COR4463.

7. Nouveau médicament selon la revendication 3 utile pour le traitement des états dépressifs.

8. Composition pharmaceutique caractérisée en ce qu'elle contient à titre de principe actif au moins un produit selon la revendication 1 en association avec un véhicule pharmaceutique ou un excipient approprié.

## Revendications (Revendications pour l'(les) Etat(s) contractant(s) suivant(s): GR)

1. Nouveaux produits de formule générale 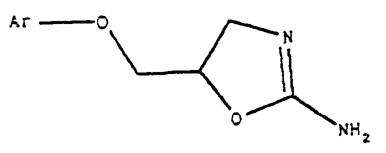 avec Ar = phényl-2 phényl ou phényl-3 phényl ou phényl-4 phényl.

2. Méthode de préparation des produits selon la revendication 1, caractérisée en ce que l'on condense dans un solvant organique soluble dans l'eau tel que les alcools inférieurs, le tétrahydrofuranne, le diméthylformamide et à une température comprise entre 0° et 80° , le dérivé mono sodé du cyanamide sur un aryloxy-1 époxy-2,3 propane de formule: 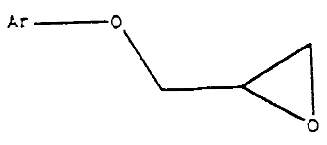 avec Ar = phényl-2 phényl ou phényl-3 phényl ou phényl-4 phényl.

## Revendications (Revendications pour l'(les) Etat(s) contractant(s) suivant(s): ES)

1. Méthode de préparation de produits de formule générale 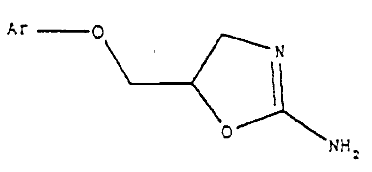 avec Ar = phényl-2 phényl ou phényl-3 phényl ou phényl-4 phényl, caractérisée en ce que l'on condense dans un solvant organique soluble dans l'eau tel que les alcools inférieurs, le tétrahydrofuranne, le diméthylformamide et à une température comprise entre 0° et 80° , le dérivé mono sodé du cyanamide sur un aryloxy-1 époxy-2,3 propane de formule:

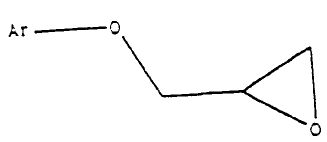

## Claims (Claims for the following Contracting State(s): AT, BE, CH, DE, DK, FR, GB, IT, LI, LU, NL, SE)

1. Novel products having the general formula : 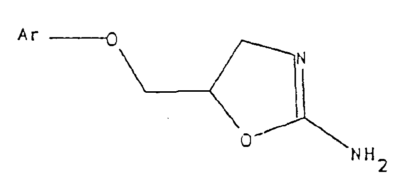 wherein Ar is phenyl-2 phenyl or phenyl-3 phenyl or phenyl-4 phenyl.

2. Method of preparing products according to claim 1, characterized in that one condenses, in an organic solvent which is soluble in water such as the lower alcohols, the tetrahydrofuran, the dimethylformamide, and at a temperature comprised between 0° and 80°C, the monosodium derivate of cyanamide on an aryloxy-1 epoxy-2,3 propane having formula : 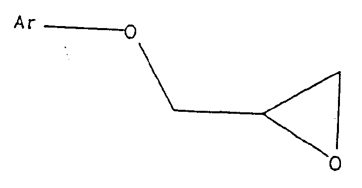 wherein Ar is phenyl-2 phenyl or phenyl-3 phenyl or phenyl-4 phenyl.

3. Novel medicine characterized in that the active principle is constituted by at least one of the products according to claim 1.

4. Novel medecine according to claim 3, characterized in that the active principle is constituted by amino-2 (phenyl-2 phenoxymethyl)-5 oxazoline or COR3264.

5. Novel medecine according to claim 3, characterized in that the active principle is constituted by amino-2 (phenyl-4 phenoxymethyl)-5 oxazoline or COR4408.

6. Novel medecine according to claim 3, characterized in that the active principle is constituted by amino-2 (phenyl-3 phenoxymethyl)-5 oxazoline or COR4463.

7. Novel medecine according to claim 3, useful for treating the depressive states.

8. Pharmaceutical composition characterized in that it contains as active principle at least one of the products according to claim 1 in association with appropriate pharmaceutical carrier or excipient.

## Claims (Claims for the following Contracting State(s): GR)

1. Novel products having the general formula : 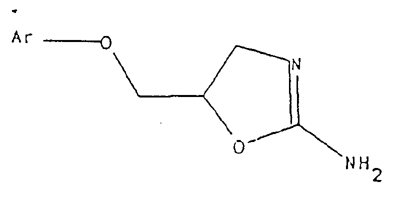 wherein Ar is phenyl-2 phenyl or phenyl-3 phenyl or phenyl-4 phenyl.

2. Method of preparing products according to claim 1, characterized in that one condenses, in an organic solvent which is soluble in water such as the lower alcohols, the tetrahydrofuran, the dimethylformamide, and at a temperature comprised between 0° and 80°C, the monosodium derivate of cyanamide on an aryloxy-l epoxy-2,3 propane having formula : 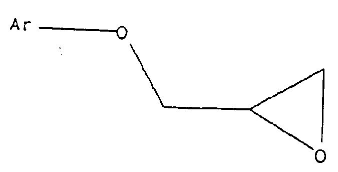 wherein Ar is phenyl-2 phenyl or phenyl-3 phenyl or phenyl-4 phenyl.

## Claims (Claims for the following Contracting State(s): ES)

1. Method of preparing products having the general formula : 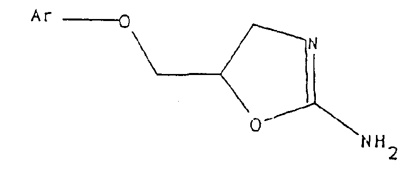 wherein Ar is phenyl-2 phenyl or phenyl-3 phenyl or phenyl-4 phenyl characterized in that one condenses, in an organic solvent which is soluble in water such as the lower alcohols, the tetrahydrofuran, the dimethylformamide, and at a temperature comprised between 0° and 80°C, the monosodium derivate of cyanamide on an aryloxy-1 epoxy-2,3 propane having formula :

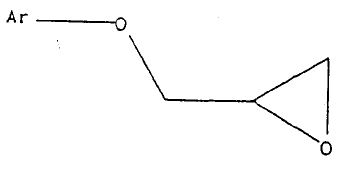

## Patentansprüche (Patentansprüche für folgende(n) Vertragsstaat(en): AT, BE, CH, DE, FR, GB, IT, LI, LU, NL, SE)

1. Neue Produkte der allgemeinen Formel 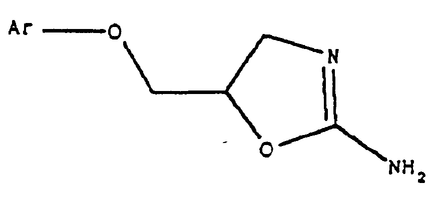 mit Ar = Phenyl-2-Phenyl, Phenyl-3-Phenyl oder Phenyl-4-Phenyl

2. Verfahren zur Herstellung von Produkten nach Anspruch 1, dadurch gekennzeichnet, daß man in einem in Wasser löslichen organischen Lösungsmittel wie niedere Alkohole, Tetrahydrofuran, Dimethylformamid und bei einer Temperatur von 0 °C bis 80 °C das Mononatrium-Derivat des Cyanamids mit einem Aryloxy-1-epoxy-2,3-propan der Formel 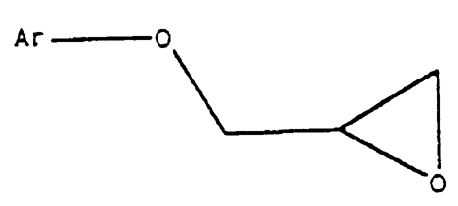 mit Ar = Phenyl-2-Phenyl, Phenyl-3-Phenyl oder Phenyl-4-Phenyl kondensiert.

3. Neues Medikament, dadurch gekennzeichnet, daß die aktive Substanz aus mindestens einem der Produkte nach Anspruch 1 besteht.

4. Neues Medikament nach Anspruch 3, dadurch gekennzeichnet, daß die aktive Substanz aus 2-Amino-5-(2-phenyl-phenoxy-methyl)-oxazolin oder COR3264 besteht.

5. Neues Medikament nach Anspruch 3, dadurch gekennzeichnet, daß die aktive Substanz aus 2-Amino-5-(4-phenyl-phenoxy-methyl)-oxazolin oder COR4408 besteht.

6. Neues Medikament nach Anspruch 3, dadurch gekennzeichnet, daß die aktive Substanz aus 2-Amino-5-(3-phenyl-phenoxy-methyl)-oxazolin oder COR4463 besteht.

7. Neues Medikament nach Anspruch 3, das für die Behandlung von Depressionszuständen geeignet ist.

8. Pharmazeutische Zusammensetzung, dadurch gekennzeichnet, daß sie als aktive Substanz mindestens ein Produkt nach Anspruch 1, in Verbindung mit einem pharmazeutischen Träger oder einer geeigneten Grundmasse enthält.

## Patentansprüche (Patentansprüche für folgende(n) Vertragsstaat(en): GR)

1. Neue Produkte der allgemeinen Formel 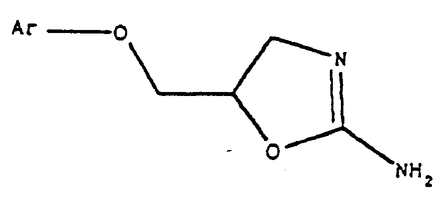 mit Ar = Phenyl-2-Phenyl, Phenyl-3-Phenyl oder Phenyl-4-Phenyl

2. Verfahren zur Herstellung von Produkten nach Anspruch 1, dadurch gekennzeichnet, daß man in einem in Wasser löslichen organischen Lösungsmittel wie niedere Alkohole, Tetrahydrofuran, Dimethylformamid und bei einer Temperatur von 0 °C bis 80 °C das Mononatrium-Derivat des Cyanamids mit einem Aryloxy-1-epoxy-2,3-propan der Formel 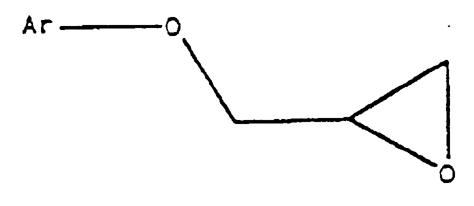 mit Ar = Phenyl-2-Phenyl, Phenyl-3-Phenyl oder Phenyl-4-Phenyl kondensiert.

## Patentansprüche (Patentansprüche für folgende(n) Vertragsstaat(en): ES)

1. Verfahren zur Herstellung von Produkten der allgemeinen Formel: 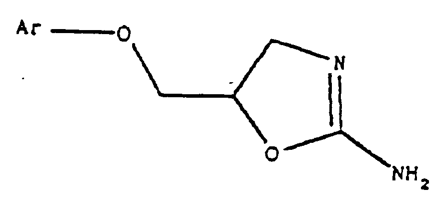 mit Ar = Phenyl-2-Phenyl, Phenyl-3-Phenyl oder Phenyl-4-Phenyl, dadurch gekennzeichnet, daß man in einem in Wasser löslichen organischen Lösungsmittel wie niedere Alkohole, Tetrahydrofuran, Dimethylformamid und bei einer Temperatur von 0 °C bis 80 °C das Mononatrium-Derivat des Cyanamids mit einem Aryloxy-1-epoxy-2,3-propan der Formel 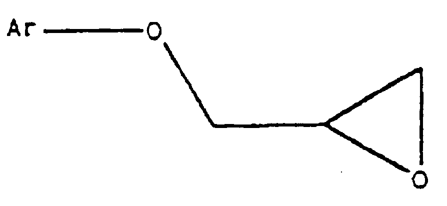 kondensiert.
